(19) **Europäisches Patentamt / European Patent Office / Office européen des brevets**

(11) **EP 1 510 217 B1**

(12) **EUROPEAN PATENT SPECIFICATION**

(45) Date of publication and mention
of the grant of the patent:
**11.11.2015 Bulletin 2015/46**

(51) Int Cl.:
***A61P 29/00*** *(2006.01)*

(21) Application number: **02724085.2**

(22) Date of filing: **09.04.2002**

(86) International application number:
**PCT/CN2002/000246**

(87) International publication number:
**WO 2003/086425 (23.10.2003 Gazette 2003/43)**

(54) **AN ANTI-RHEUMATISM MEDICAMENT AND METHOD FOR ITS PREPARATION**

ANTIRHEUMATIKUM UND VERFAHREN ZU SEINER HERSTELLUNG

MEDICAMENT CONTRE LES RHUMATISMES ET PROCEDE DE PRODUCTION

(84) Designated Contracting States:
**AT BE CH CY DE DK ES FI FR GB GR IE IT LI LU MC NL PT SE TR**

(43) Date of publication of application:
**02.03.2005 Bulletin 2005/09**

(73) Proprietors:
- **Sichuan Institute of Chinese Materia Medica Sichuan 610041 (CN)**
- **Guangzhou Chen Li Ji Pharmaceutical Factory Guangdong 510290 (CN)**

(72) Inventor: **DENG, Wenlong Sichuan 610041 (CN)**

(74) Representative: **Groth & Co. KB P.O. Box 6107 102 32 Stockholm (SE)**

(56) References cited:
**CN-A- 1 142 970     CN-A- 1 178 697**

- **ZHNAG CUN: 'A treatment of rheumatic arthritis with tripterygium hypoglaucum' HUNAN MAGAZINE OF TRADITIONAL CHINESE MEDICINE vol. 4, no. 4, 1988, pages 15 - 16, XP008168459**

## Description

FIELD OF THE INVENTION

The present invention is concerned with a pharmaceutical composition for treating rheumatism and the preparation thereof.

BACKGROUND OF THE INVENTION

It is believed that the rheumatoid arthritis (RA) is refractory and about 18,000,000 RA patients have been disabled because of this disease. The medicine research for curing RA has continued about a century. Aspirin is the first medicine which is widely used to treat RA. The medicine to treat RA can be divided into 2 kinds: non-steroidal anti-inflammatory drugs (NSAIDs) and immunosuppressive agent. NSAIDs include cyclophthasine, antinfan and adrenal cortex hormone. The clinical researches have proved the effectiveness of NSAIDs. The immunosuppressive agent includes methotrexate, cyclophosphane, penicillamine and et al. The regulation of the immune system has become one of the important therapies in the recent years. But all the medicines which are used to treat rheumatism have serious side-effect. The medicine which can treat rheumatism effectively and non-poisonously hasn't been invented by now.

There are 3 directions in the research of anti-rheumatic medicine that should be emphasized. The first direction is NSAIDs and cytokine antagonist, such as recombined soluble INF antagonist, IL-1 inhibitor and PAF inhibitor. The second direction is the new immunosuppressive agent and immunomodulator, such as cyclosporin A. The third direction is the compound medicines.

In the TCM, the research on the "arthralgia disease" (equals to the definition of rheumatism in the modem medicine) can be traced back to the Han dynasty more than 1,500 years ago. Three prescriptions: "Ma Xing Shi Gan decoction", "Fangji Huangqi decoction" and "Wutou decoction", which is used to treat "Bi Zheng" were recorded in the medicine classics "Shanghan Lun" written by the famous doctor Zhang Zhongjing at that time. *Gelsemium elegans* Benth is a kind of wild plant in Sichuan province and it has been proved to be effective to treat rheumatism in a clinical research carried at the local area. But the further study found that it had a serious side-effect on the reproduction organs and some other uncontrollable problem.

The treatment of "arthralgia disease" by the method of TCM has reached a high level by numerous doctors' development in so long a history. By now, there are many effective prescriptions and herbs. There are more than 80 kinds of herbs and 29 kinds of patent medicines recorded in Chinese pharmacopoeia 1995 edition and 2000 edition.

Zhang Cun described in 'A treatment of rheumatic arthritis with Tripterygium hypoglaucum' published in HUNAN MAGAZINE OF TRADITIONAL CHINESE MEDICINE vol. 4, no. 4, 1988, pages 15 - 16, that *Tripterygium hypoglaucum* is effective for treating rheumatic arthritis in 74 clinical cases where the patient age ranges from 15 to 85 years.

But there are still many problems for example: (1) the effect is not good enough in treating the serious arthralgia disease such as rheumatoid arthritis; (2) the dosage forms are not fit for the modem life. (3) some medicine has good effect, but the side-effect is serious too, such as the extract of *Triperygium wilfordii*. Therefore, it is necessary to develop anti-rheumatic medicine with high efficiency and low toxicity which is also convenient for administration. This medicine should have similar effect and lower side effect compared with the synthetic anti-rheumatic medicine.

SUMMARY OF THE INVENTION

Accordingly, the present invention provides a pharmaceutical composition suitable for treating rheumatism as defined in claim 1.

The present invention further provides a method for preparing the pharmaceutical composition as defined in claim 3 or 4.

The present invention also relates to the use of the pharmaceutical composition in the manufacture of a medicament for use in treating rheumatism as defined in claim 8.

DISCLOSURE OF THE INVENTION

The invention is to provide an anti-rheumatic medicine with high efficiency and low toxicity which is also convenient for administration, and the preparation thereof.

The invented medicine's technical proposal is realized by using the crude herbs as follows:

*Tripterygium hypoglaucum* (Levl.) Hutch.
*Epimedium brevicornum* Maxim.
*Lycium barbarum* L.

*Cuscuta chinensis* Lam. (or *Cuscuta australis* R. Br.)

The invented medicine is made from the crude herbs above.

The ratio of crude herbs of the material is as follows:

| | |
|---|---|
| *Tripterygium hypoglaucum* (Levl.) Hutch. | 1-4 parts by weight |
| *Epimedium brevicornum* Maxim. | 1-4 parts by weight |
| *Lycium barbarum* L. | 1-4 parts by weight |
| *Cuscuta chinensis* Lam. | 1-4 parts by weight |

The other optimal ratios of crude herbs of the material is as follows:

| | |
|---|---|
| *Tripterygium hypoglaucum* (Levl.) Hutch. | 2 parts by weight |
| *Epimedium brevicornum* Maxim. | 2 parts by weight |
| *Lycium barbarum* L. | 1 part by weight |
| *Cuscuta chinensis* Lam. | 1 part by weight |

The crude herbs are prepared in said ratios and then they can be made into any dosage forms used in the clinic, such as the bolus form, the powder forms, the ointment forms, the tablet forms, the soft or hard capsule forms, the granule forms, the injection forms and so on.

The preparation method of the invented medicine is as follows.

The crude herbs are prepared in weight ratios:

| | |
|---|---|
| *Tripterygium hypoglaucum* (Levl.) Hutch | 1-4 parts by weight |
| *Epimedium brevicornum* Maxim | 1-4 parts by weight |
| *Lycium barbarum* L | 1-4 parts by weight |
| *Cuscuta chinensis* Lam | 1-4 parts by weight |

The *Tripterygium hypoglaucum* (Levl.) Hutch. and *Epimedium brevicornum* Maxim are smashed. Then the powders are decocted by water for 2 ~ 4 times separately. The *Lycium barbarum* L and *Cuscuta chinensis* Lam are soaked in the hot water (80~95°C ) for 1 ~ 3 times separately. The decocted fluid and the immersion fluid of the herbs are collected and added to the corresponding macroporous adsorbent resin column separately. After the adsorption, the columns are washed until the washing solution turns clear. Then the columns are eluted with 60%-80% alcohol. The eluates are collected while the colour turns from a deep colour to a weak colour. Then the alcohol in the upper part of the column is pushed out by high pressure water and mixed with the eluate. The mixed eluate is 3 ~ 8 times heavier than the corresponding crude herb. All the four eluates are recovered and condensed separately to the specific density of 1.10. The condensed eluates are dehydrated by spray drying to get the extract of the crude herbs. The four kinds of extracts are mixed uniformly to be made into any dosage forms as required by the clinic.

The optimal preparation method of the invented medicine is as follows.

The crude herbs are prepared in weight ratios:

| | |
|---|---|
| *Tripterygium hypoglaucum* (Levl.) Hutch | 2 parts by weight |
| *Epimedium brevicornum* Maxim | 2 parts by weight |
| *Lycium barbarum* L | 1 part by weight |
| *Cuscuta chinensis* Lam | 1 part by weight |

The *Tripterygium hypoglaucum* (Levl.) Hutch. is smashed. Then the powder is decocted three times with 13-, 10- and 10-fold weight of water respectively. Each time is 1 hour. The *Epimedium brevicornum* Maxim is cut into piece. Then the herb pieces are decocted three times with 15-, 10-, 10-fold weight of water respectively. Each time is 1 hour. The *Lycium barbarum* L is smashed to crude powder and soaked in 20-fold weight of water at 80°C for 3 times. Each time is 1 hour. The *Cuscuta chinensis* Lam is smashed to crude powder and soaked in 31-fold weight of water at 80°C for 3 times. Each time is 1 hour. The decocted fluid and the immersion fluid of the herbs are filtrated separately and added to the corresponding macroporous adsorbent resin column JD-1 (WLD). After the adsorption, the columns are eluted with 70% alcohol. The eluates are collected while the color turns from a deep colour to a very weak colour. The alcohol is recovered from the eluate. Then the remaining eluate is condensed and dehydrated to get the extract powder. The four kinds of extract powders are mixed uniformly to be made into any dosage forms as required by the clinic.

The invented medicine can be prepared by the method as follows:

The crude herbs are prepared in weight ratios as mentioned before. The *Tripterygium hypoglaucum* (Level.) Hutch. and *Epimedium brevicornum* Maxim are cut into pieces. The *Lycium barbarum* L and *Cuscuta chinensis* Lam are crushed or not. The 4 kind of herbs are extracted in the 0-95% alcohol at 10-98°C for 1-4 times separately or together. The alcohol is recovered from the extracts separately or together. Then the extracts are condensed, dehydrated, smashed and mixed uniformly. The mixed powder can be made into any dosage form as required by the clinic.

The invented medicine is named as Fengshiping Capsule. It has been proved by the pharmacodynamics research that Fengshiping Capsule could inhibit the primary and secondary injury adjuvant arthritis (AA). It could inhibit the delayed hypersensitivity (DTH) in the ear of the mouse caused by the 2,4 dinitrofluorobenzene (DNFB). It could inhibit the production of anti-hemolysin antibodies and the activity of IL-1, IL-2, IL-6 and TNF in macrophages and splenocytes. The Fengshiping Capsule could inhibit lymphocyte transformation induced by ConA. It could inhibit $CD_4$ and $CD_8$ cells remarkably, especially $CD_4$ cells, but the ratio of $CD_4/CD_8$ was not affected very much. There was a remarkable linear relationship between the dosage and the effect. 12-18g (crude medicine)/kg is the minimum effective dose. The invented medicine could inhibit the activity of NK cells. In the effective dose, Fengshiping Capsule does not cause the atrophy of the important immune organs such as thymus and spleen, and did not inhibit the phagocytic activity of the macrophage.

The invented medicine had a remarkable anti-inflammatory action. It could inhibit the over penetrating condition of capillary in the mouse's abdominal cavity caused by the injected ethanoic acid. It may improve the swelling in the ear of the mouse caused by the croton oil. It may inhibit pleuritis in mouse and the assembling of WBC in the CMC cyst of rat induced by the carrageenan. But the Fengshipng Capsule couldn't inhibit rat's foot swelling induced by carrageenan and granuloma caused by tampon obviously. The Fengshipng Capsule could inhibit the body-twist reaction caused by ethanoic acid in the mouse remarkably.

Experimental example 1: the effect on the adjuvant arthritis (AA)

1.1 The preventing effect on the AA of the invented medicine

72 isogenic SD rats of the same batch, half male and half female, 180 -- 220g weight, were divided randomly into 6 groups. Each group has 12 rats. Every 6 rats lived in a cage. The perimeter of both anklejoints and feet of the rat were measured accurately and recorded as the normal value. All the rats were fed with the same volume of the invented medicine on the corresponding concentration or the solution of Xihuangqi (i.e. tragacanth) as control by gastric intubation. 1 hour later, all the rats were injected with 0.1ml Freund's complete adjuvant (FCA) under the skin of the left hindfoot pad. In the next 30 days, all the rats were fed with the corresponding medicine once a day on the same dosage. And in these days, the rats were measured of the perimeters of both anklejoints and feet once a day. In this experiment, the swelling degree ($\Delta$ cm) is equal to the difference value of the perimeters measured after the FCA injection and before the FCA injection. (See the result in table 1.1 and 1.2) At the end of the experiment, the major organs of the rats were weighed. (See the table 1.3, 1.4)

**Table 1.1 The effect of the Fengshiping on the swelling degree of the left anklejoint and foot after the injection of FCA in the rat AA model ($\overline{X} \pm S$)**

| Group | Dose (g/kg) | Swelling degree ($\triangle$cm) | | | | | | |
|---|---|---|---|---|---|---|---|---|
| | | 1d | 2d | 3d | 9d | 12d | 14d | 16d |
| Control | - | 0.69±0.17 | 0.69±0.12 | 0.92±0.18 | 0.84±0.41 | 1.10±0.30 | 1.65±0.68 | 2.10±0.55 |
| Fengshiping | 7.5 | 0.74±0.12 | 0.66±0.074 | 0.83±0.13 | 0.77±0.27 | 1.11±0.45 | 1.34±0.53 | 1.91±0.61 |
| Fengshiping | 15 | 0.80±0.24 | 0.62±0.13 | 0.76±0.18 | 0.49±0.17* | 0.73±0.34* | 1.00±0.48* | 1.38±0.67* |
| Fengshiping | 30 | 0.75±0.19 | 0.67±0.19 | 0.87±0.28 | 0.63±0.22 | 0.73±0.34* | 0.82±0.43** | 1.05±0.53** |
| Tripterygium hypoglaucum (Levl.) Hutch. | 5 | 0.72±0.11 | 0.68±0.16 | 0.91±0.18 | 0.66±0.23 | 0.88±0.29 | 1.03±0.36* | 1.37±0.33* |
| prednisone | 0.01 | 0.64±0.14 | 0.64±0.16 | 0.50±0.26 | 0.46±0.25 | 0.72±0.46* | 0.87±0.46** | 1.28±0.69* |

| Group | Dose (g/kg) | Swelling degree($\triangle$cm) | | | | | | |
|---|---|---|---|---|---|---|---|---|
| | | 18d | 20d | 22d | 24d | 26d | 28d | |
| Control | - | 2.18±0.44 | 2.05±0.46 | 2.00±0.46 | 2.04±0.57 | 1.92±0.65 | 1.83±0.67 | |
| Fengshiping | 7.5 | 1.74±0.73 | 1.81±0.55 | 1.81±0.52 | 1.77±0.55 | 1.65±0.55 | 1.55±0.49 | |
| Fengshiping | 15 | 1.32±0.59** | 1.28±0.58** | 1.34±0.61* | 1.33±0.67* | 1.20±0.64* | 1.08±0.58** | |
| Fengshiping | 30 | 0.95±0.50** | 0.87±0.51** | 0.95±0.54** | 0.89±0.59* | 0.90±0.57** | 0.86±0.51** | |
| Tripterygium hypoglaucum (Levl.) Hutch. | 5 | 1.47±0.43** | 1.50±0.43** | 1.49±0.43* | 1.42±0.53* | 1.40±0.56* | 1.32±0.57 | |
| prednisone | 0.01 | 1.18±0.7**6 | 1.03±0.67*` | 1.05±0.69* | 0.90±0.64** | 0.86±0.65** | 0.85f0.59** | |

Comparing to the control group *P<0.05, **P<0.01 (the signs have the same meaning in the following tables)

EP 1 510 217 B1

**1.2 The effect of the Fengshiping on the swelling degree of the left anklejoint and footafter the injection of FCA in the rat AA model ($\overline{X} \pm S$)**

| Group | Dose (g/kg) | Swelling degree ($\triangle$cm) | | | | | |
|---|---|---|---|---|---|---|---|
| | | 2d | 9d | 12d | 14d | 16d | 18d |
| Control | - | 0.14±0.05 | 0.06±0.10 | 0.34±0.36 | 0.80±0.52 | 1.43±0.67 | 1.36±0.61 |
| Fengshiping | 7.5 | 0.18±0.06 | 0.10±0.14 | 0.26±0.36 | 0.82±0.52 | 1.31±0.64 | 1.28±0.71 |
| Fengshiping | 15 | 0.15±0.08 | 0.02±0.06 | 0.13±0.10* | 0.37±0.31* | 0.90±0.56* | 0.79±0.60* |
| Fengshiping | 30 | 0.18±0.09 | 0.06±0.06 | 0.16±0.08* | 0.29±0.20** | 0.49±0.41** | 0.33±0.29** |
| Tripterygium hypoglaucum (Levl.) Hutch. | 5 | 0.16±0.07 | 0.01±0.07 | 0.11±0.10 | 0.44±0.19** | 0.87±0.56* | 0.84±0.67* |
| prednisone | 0.01 | 0.20±0.06 | 0.08±0.08 | 0.21±0.16 | 0.44±0.43 | 0.99±0.63 | 0.84±0.74* |

| Group | Dose (glkg) | Swelling degree ($\triangle$m) | | | | |
|---|---|---|---|---|---|---|
| | | 20d | 22d | 24d | 26d | 28d |
| Control | | 1.28±0.57 | 1.38±0.64 | 1.35±0.75 | 1.20±0.78 | 1.12±0.63 |
| Fengshiping | 7.5 | 1.33±0.71 | 1.31±0.73 | 1.27±0.73 | 1.16±0.73 | 1.07±0.65 |
| Fengshiping | 15 | 1.74±0.57* | 1.92±0.61* | 0.95±0.64* | 0.88±0.58* | 1.83±0.55 |
| Fengshiping | 30 | 0.27±0.30** | 0.34±0.31** | 0.32±0.33** | 0.31±0.32** | 0.34±0,32** |
| Tripterygium hypoglaucum (Levl.) Hutch. | 5 | 0.82±0.65* | 0.89±0.70* | 0.80±0.67* | 0.83±0.68 | 0.75±0.69 |
| prednisone | 0.01 | 0.82±0.72* | 0.79±0.74* | 0.75±0.67** | 0.68±0.64* | 0.71±0.67 |

EP 1 510 217 B1

**1.3 The effect of the Fengshipng on the body weight of the AA rats ($\bar{X} \pm S$)**

| Group | Dose (g/kg) | Body weight change(g) | | |
|---|---|---|---|---|
| | | Initiative BW | BW at 1 month later | BW change |
| Control | - | 228±34 | 231±52 | 3 |
| Fengshiping | 7.5 | 229±34 | 220±46 | -9 |
| Fengshiping | 15 | 223±40 | 232±34 | 9 |
| Fengshiping | 30 | 224±37 | 256±60 | 32 |
| Tripterygium hypoglaucum (Levl.) Hutch. | 5 | 226±45 | 230±43 | 4 |
| prednisone | 0.01 | 264±55 | 244±31 | -21 |

**1.4 The effect of the Fengshiping on the organ weight of the immune system in the AA rats (prevention experiment)($\bar{X} \pm S$)**

| Group | Dose (g/kg) | Organ index [(organ weight/body weight)/100] | | | |
|---|---|---|---|---|---|
| | | Liver | Spleen | Thymus | adrenal gland |
| Control | - | 3.92±0.65 | 0.34±0.10 | 0.098±0.040 | 0.027±0.01 |
| Fengshiping | 7.5 | 3.73±0.29 | 0.31±0.09 | 0.078±0.038 | 0.027±0.008 |
| Fengshiping | 15 | 3.48±0.32 | 0.38±0.10 | 0.100±0.034 | 0.023±0.005 |
| Fengshiping | 30 | 3.38±0.28* | 0.44±0.12* | 0.100±0.032 | 0.022±0.007 |
| Tripterygium hypoglaucum (Levl.) Hutch. | 5 | 3.21±0.30** | 0.36±0.05 | 0.052±0.011** | 0.026±0.009 |
| prednisone | 0.01 | 3.04±0.20** | 0.32±0.08 | 0.050±0.060** | 0.020±0.004* |

1.2 The therapeutic effect on the AA of the invented medicine

50 male SD rats were divided into 5 groups at random. The rats were treated in the same manner as those for the prevention experiment, but the corresponding medicines were fed 13 days after the injection of the FCA. The medicines were fed once a day for 2 weeks. The swelling degree (Δ cm) was the difference of the perimeters between the value of first administration day and the other days. (See the result in table 1.5, 1.6) The major organs' weight is shown in table 1.7.

**1.5 The therapeutic effect of Fengshiping on the swelling degree of The left anklejoint and foot in the AA rats ($\bar{X} \pm S$)**

| Group | Dose (g/kg) | Swelling degree (Δcm) | | | |
|---|---|---|---|---|---|
| | | 1d | 2d | 4d | 6d |
| Control | - | 1.81±0.27 | 1.92±0.19 | 2.12±0.22 | 2.16±0.27 |
| Fengshiping | 7.5 | 1.68±0.50 | 1.64±0.54 | 1.70±0.57 | 1.82±0.61 |
| Fengshiping | 15 | 1.44±0.41* | 1.51±0.36** | 1.65±0.34** | 1.74±0.31** |
| Fengshiping | 30 | 1.50±0.56 | 1.48±0.41** | 1.51±0.44** | 1.59±0.51** |
| prednisone | 0.01 | 1.78±0.51 | 1.70±0.51 | 1.63±0.50* | 1.58±0.50** |

| Group | Dose (g/kg) | Swelling degree (Δcm) | | | |
|---|---|---|---|---|---|
| | | 8d | 10d | 12d | 14d |
| Control | - | 1.92±0.32 | 1.87±0.34 | 1.92±0.39 | 1.78±0.44 |
| Fengshiping | 7.5 | 1.67±0.68 | 1.60±0.71 | 1.61±0.77 | 1.58±0.71 |
| Fengshiping | 15 | 1.46±0.37** | 1.48±0.30* | 1.28±0.37** | 1.22±0.38** |
| Fengshiping | 30 | 1.29±0.58** | 1.29±0.65** | 1.26±0.67** | 1.20±0.68* |
| prednisone | 0.01 | 1.27±0.46** | 1.09±0.54** | 0.94±0.50** | 0.94±0.42** |

**1.6 The therapeutic effect of Fengshiping on the swelling degree of the right anklejoint and foot in the AA rats**
$$(\bar{X} \pm S)$$

| Group | Dose (g/kg) | Swelling degree ($\triangle$cm) | | | |
|---|---|---|---|---|---|
| | | 2d | 4d | 6d | 8d |
| Control | - | 0.36±0.26 | 0.45±0.25 | 0.55±0.34 | 0.47±0.29 |
| Fengshiping | 7.5 | 0.12±0.25 | 0.34±0.32 | 0.48±0.41 | 0.28±0.38 |
| Fengshiping | 15 | 0.21±0.18 | 0.38±0.27 | 0.44±0.33 | 0.21±0.33* |
| Fengshiping | 30 | 0.10±0.48 | 0.06±0.28** | 0.11±0.24** | 0.06±0.27** |
| prednisone | 0.01 | 0.10±0.13* | 0.15±0.28* | 0.11±0.25** | 0.08±0.34** |

| Group | Dose (g/kg) | Swelling degree($\triangle$cm) | | |
|---|---|---|---|---|
| | | 10d | 12d | 14d |
| Control | - | 0.48±0.25 | 0.46±0.31 | 0.40±0.36 |
| Fengshiping | 7.5 | 0.35±0.30 | 0.30±0.29 | 0.30±0.35 |
| Fengshiping | 15 | 0.19±0.45* | 0.06±0.31** | -0.06±0.34** |
| Fengshiping | 30 | 0.02±0.39** | 0.05±0.38* | -0.02±0.41** |
| prednisone | 0.01 | -0.13±0.28** | -0.26±0.36** | -0.33±0.39** |

n=10, comparing with the control group, *P<0.05, **P<0.01

**1.7 The effect of the Fengshiping on the organ weight of the immune system in the AA rats ($\bar{X} \pm S$)**

| Group | Dose (g/kg) | Organ index [(organ weight/body weight)/100] | | | |
|---|---|---|---|---|---|
| | | Liver | Spleen | Thymus | adrenal gland |
| Control | - | 0.35±0.23 | 0.3 ± 0.061 | 0.073 ± 0.014 | 0.026 ± 0.0071 |
| Fengshiping | 7.5 | 3.21 ± 0.52 | 0.3 ± 0.091 | 0.071 ± 0.026 | 0.024 ± 0.0085 |
| Fengshiping | 15 | 3.40 ± 0.54 | 0.36 ± 0.014 | 0.067 ±0.022 | 0.023 ±0.0048 |
| Fengshiping | 30 | 2.79 ± 0.43 | 0.32 ± 0.083 | 0.069 ±0.029 | 0.023 ±0.0072 |
| Tripterygium hypoglaucum (Levl.) Hutch. | 5 | 3.92 ± 0.59 | 0.35 ± 0.100 | 0.075 ± 0.034 | 0.027 ± 0.0060 |
| prednisone | 0.01 | 3.52 ± 0.35 | 0.28 ± 0.047* | 0.05 ± 0.011** | 0.02 ± 0.0043* |

The data showed in the table 1.1, 1.2, 1.3, 1.5 and 1.6 proved that the Fengshiping could strongly inhibit the primary and secondary injury caused by FCA, whenever the medicine was fed at the beginning of the FCA injection or 2 weeks after the FCA injection. The experiments proved that the Fengshiping had both the preventing and the therapeutic effect. By comparing the effect of Fengshiping on the swelling degree in the anklejoint and foot, we found that the Fengshiping could inhibit the specific immunoswelling in the anklejoint better than the nonspecific immunoswelling in the foot of rats. This result indicated that the main effect of Fengshiping was inhibiting the immunity inflammatory reaction.

The data in the table 1.3, 1.4 and 1.7 showed that the AA rats had no obvious BW increase during the period of the experiment. In the group fed with the Fengshiping on the effective dosage, the rats still had BW increase. In the groups of prednisone and preventing, the BW of rats had decreased, while the thymus and adrenal gland were atrophied. In the group of *Tripterygium hypoglaucum* (Levl.) Hutch, the thymus had shrunk. But in the 3 groups fed with the different dosage of Fengshiping, no atrophy of the thymus and adrenal gland were observed.

1.3 The pathologic change of the AA after the treatment of the invented medicine in rats

45 SD rats, 180±20g weight, were divided into 6 groups. After the AA caused by FCA appeared, all the rats were fed with Fengshiping solution by gastric intubation for 5 days once a day. 1 hour after the last administration, the joint index of the rats was measured and calculated. The secondarily injured hindlimb joints after the FCA injection were taken off and soaked in the formaldehyde. Afterwards the tissues were HE tinted, the pathological change of the synovium and cartilage were observed and recorded. The data were showed in table 1.8.

**1.8 The effect of Fengshiping on the AA joint index in the rats ($\overline{X} \pm S$)**

| Group | Dose (g/kg) | Rat number | Joint index |
|---|---|---|---|
| Control | - | 8 | 0** |
| AA model | - | 7 | 6.2 ± 0.49 |
| Fengshiping | 7.5 | 9 | 4.86 ± 0.90** |
| Fengshiping | 15 | 7 | 4.71 ± 0.95** |
| Fengshiping | 30 | 7 | 4.56 ± 1.13** |
| Glucosidorum Tripterygll Totorum | 0.006 | 7 | 4.57 ± 0.79** |

**Comparing with the model group**$**P<0.01$**

The joint index was the sum of the inflammatory scores of the four limbs. According to the degree of inflammation, each limb was evaluated on the criteria as following: normal (0), red without swelling (1), red and swelling (2), seriously swelling (3), deforming and tetanus (4).

Observed from the microscope, the joint synovial membranes of the rat hindlimb were hyperplasia in the model group; and the collagen fiber had increased; there was infiltration of lymphocytes and plasma cells in the tissue. The obvious granuloma had formed. The synovial cells had degenerated and the cytochylema had been tinted red; karyopyknosis were observed; the epithelium had exfoliated in some part of the synovial membrane. The cartilage turned atrophy; the surface of it was rough and some of the chondrocytes had proliferated lightly.

After the treatment of the Fengshiping, the inflammation of the joint synovial membrane was inhibited, more collagen fiber was produced; less synovial cells exfoliated; the cells on the surface of the cartilage had proliferated and the surface had turned smooth. The cartilage was on the recovering condition.

Experimental example 2: The effect of Fengshiping on the delayed hypersensitivity reaction (DTH) caused by 2,4-DNFB in the ear of the mouse 50 NIH mice, half male and half female, were divided into 5 groups. Each mouse was led to hypersensitivity reaction by using the 1% DNFB acetone solution on the dosage of 0.025ml at the right place of the abdomen where the pilus had been cut yet. Using the same solution on the same place was to enhance the hypersensitivity reaction on the third day. On the fifth day, all the mice were smeared with the 1 % DNFB edible oil solution at the mice's right ears on the dosage of 0.01 ml each. 24 hours later, all the mice were killed. Both ears of the mouse were weighted by the torsion balance and the difference of the 2 ears was recorded as the DTH degree caused by the DNFB. The experiment was carried out on different immune and administration processes.

2.1 The effect on the DTH by the full course administration The immune and administration processes is as following:

**Table 2.1 The effect of Fengshiping on the DTH caused by DNFB in the NIH mouse ($\overline{X} \pm S$)**

| group | dose (g/kg) | Administration time (day) | Mice number | Percent of ear swelling | Percent of inhibition (%) | P value |
|---|---|---|---|---|---|---|
| control | | | 10 | 34.20 ± 3.77 | | |
| Fengshiping | 27 | 0~5 | 10 | 26.24 ± 3.34 | 23.3 | <0.01 |
| Fengshiping | 40 | 0~5 | 10 | 12.99 ± 4.96 | 62.0 | <0.01 |
| Fengshiping | 60 | 0~5 | 10 | 10.43 ± 7.53 | 69.5 | <0.01 |
| cortisumman | 0.003 | 0~5 | 10 | 13.93 ± 4.41 | 59.3 | <0.01 |
| control | | | 10 | 42.43 ± 5.28 | | |
| Fengshiping | 40 | -2~0 | 10 | 31.50 ± 10.52 | 25.0 | <0.01 |
| Fengshiping | 40 | -2~2 | 10 | 30.88 ± 7.92 | 27.2 | <0.01 |
| Fengshiping | 40 | -2~5 | 10 | 21.07 ± 4.62* | 50.3 | <0.01 |
| Fengshiping | 40 | 5~6 | 10 | 32.00 ± 9.37 | 41.7 | <0.01 |
| cyclophosphane | 0.05 | -2~2 | 10 | 39.40 ± 10.78 | 8.1 | >0.05 |
| cyclophosphane | 0.05 | -2~0 | 10 | 37.47 ± 6.71 | 11.7 | >0.05 |
| control | | | 10 | 38.50 ± 4.67 | | |
| cyclophosphane | 0.1×3 | 0, 2, 4 day once a day | 10 | 23.00 ± 7.65 | 40.3 | <0.01 |
| cyclophosphane | 0.25 | -3d | 10 | 41.84 ± 7.75 | -8.7 | |
| Fengshiping | 60 | 0~4 | 10 | 27.20 ± 10.20 | 29.4 | <0.01 |
| cyclophosphane+Fengshiping | 0.25+60 | -3,0~4 | 10 | 38.07 ± 6.65 | 1.1 | |

*comparing with the other groups P<0.05 或 P<0.01

According to the data showed in table 2.1, it indicated that the Fengshiping had an obvious inhibiting effect on the DTH caused by DNFB. There was a significant relationship between the dosage and the effect. The inhibiting activity increases when the dosage adds. The inhibiting percent could reach 69.5% on the dosage of 60.9g/kg.

2.2 The effect on the DTH of the different administration time

The immune and administration processes and the corresponding results had been showed in middle and bottom parts of the table 2.1. According to the results showed in the table 2.1, all the administration ways could significantly inhibit the DTH of the mouse in spite of the administration beginning from the 2 days before the sensitization and ending at the sensitization, or beginning from the 2 days before the sensitization and ending 2 days after the sensitization, or beginning from the 2 days before the sensitization and ending 5 days after the sensitization, or beginning before the attack and ending after the attack. But the administration way that began 2 days before the sensitization and ended 5 days after the sensitization had the most powerful inhibiting activity. It indicated that the Fengshiping could inhibit the DTH by a compound mechanism that it could inhibit the cells participant in the early period of the DTH, the effector cells in the advanced period and the cells related to the DTH in the middle period. This mechanism was different from that of the cyclophosphane. On a small dosage, the cyclophosphane didn't affect the DTH, if its administration way was beginning from the 2 days before the sensitization and ending at the sensitization day or 2 days after the sensitization day.

Based on the bottom part of the table 2.1, if a high dosage of cyclophosphane was fed to the mouse in one time 3 days before the sensitization, the function of the Th cells would turn hyperactive because of the powerful inhibition on the Ts cells. The DTH in the mouse would be enhanced. If the cyclophosphane was used with the Fengshiping on this administration way, it could lower the inhibiting activity of Fengshiping. This result indicated that the Fengshiping have a different mechanism to the cyclophosphane in the control of DTH. The Fengshiping may have a higher activity in inhibiting the Th cells. Experimental example 3: The effect on the humoral immunity

3.1 The effect on the production of the anti-hemolysin antibody caused by the chick RBC

190 mice, 18-22g weight, half male and half female, were divided into 19 groups. Each mouse was immunized with 5% CRBC solution 0.2 ml. The Fengshiping solutions were fed at the different times. 7 days after the immunization, all the mice were sampled the blood from the eyes. Then the blood samples were diluted and measured for the level of the anti-hemolysin antibody. The results were showed in table 3.1, 3.2 and 3.3.

**Table 3.1 The effect of Fengshiping on the produce of the hemolysin antibody in the NIH mouse ($\overline{X} \pm S$)**

| group | dose (g/kg) | Administration time | Mouse number | Hemolysin value | Inhibiting percent (%) | P value |
|---|---|---|---|---|---|---|
| control | | | 10 | 169.0 $\pm$ 62.0 | | |
| Fengshiping | 18 | 0~7 | 10 | 46.0 $\pm$ 15.6 | 72.8 | <0.01 |
| Fengshiping | 27 | 0~7 | 10 | 35.4 $\pm$ 12.0 | 79.1 | <0.01 |
| Fengshiping | 40 | 0~7 | 10 | 28.2 $\pm$ 5.9 | 83.3 | <0.01 |
| Fengshiping | 60 | 0~7 | 10 | 16.7 $\pm$ 3.0 | 90.1 | <0.01 |
| Tripterygium hypoglaucum (Levl.) Hutch. | 13.3 | 0~7 | 10 | 121.0 $\pm$ 88.0** | 28.4 | <0.015 |
| cyclophosphane | 0.02 | 0~7 | 10 | 35.0 $\pm$ 12.0 | 79.3 | <0.01 |

** comparing with the Fengshiping (40g/kg) group P<0.01

**Table 3.2 The effect of Fengshiping on the produce of the hemolysin antibody in the ICR mouse ($\overline{X} \pm S$)**

| group | dose (g/kg) | Administration time | Mouse number | Hemolysin value | Inhibiting percent (%) | P value |
|---|---|---|---|---|---|---|
| control | - | - | 10 | 124.70 $\pm$ 42.60 | | |
| Fengshiping | 12 | 0~7 | 10 | 75.00 $\pm$ 53.10 | 39.9 | <0.05 |
| Fengshiping | 18 | 0~7 | 10 | 45.60 $\pm$ 22.70 | 63.4 | <0.01 |
| Fengshiping | 27 | 0~7 | 10 | 29.10 $\pm$ 22.10 | 76.8 | <0.01 |
| Fengshiping | 40 | 0~7 | 10 | 28.20 $\pm$ 5.30 | 77.4 | <0.01 |
| Tripterygium hypoglaucum (Levl.) Hutch. | 6.0 | 0~7 | 10 | 143.50 $\pm$ 67.90** | | >0.05 |
| cyclophosphane | 0.02 | 0~7 | 10 | 27.80 $\pm$ 6.60 | 77.9 | <0.01 |

**comparing with the Fengshiping (18g/kg) group P<0.01

**Table 3.3 The effect of Fengshiping on the produce of the hemolysin antibody in the ICR mouse ($\overline{X} \pm S$)**

| group | dose (g/kg) | Administration time | Mouse number | Hemolysin value | Inhibiting percent (%) | P value |
|---|---|---|---|---|---|---|
| control | - | - | 10 | 256.0 $\pm$ 26.0 | | |
| Fengshiping | 18 | -7~7 | 10 | 198.0 $\pm$ 50.0 | 22.7 | <0.01 |
| Fengshiping | 18 | -3~7 | 10 | 156.0 $\pm$ 85.0 | 39.1 | <0.01 |
| Fengshiping | 18 | 0~7 | 10 | 98.0 $\pm$ 35.0 | 61.7 | <0.01 |

(continued)

| group | dose (g/kg) | Administration time | Mouse number | Hemolysin value | Inhibiting percent (%) | P value |
|---|---|---|---|---|---|---|
| cyclophosphane | 0.02 | 0~7 | 10 | 25.0±4.0 | 90.2 | <0.01 |

According to the data in table 3, it indicated that the Fengshiping had a remarkable inhibiting effect on the production of the anti-hemolysin antibody in the different mouse species and this effect would increase along with the increase of the dosage. There was a certain relationship between the dosage and the effect. The lowest effective dosage was 12g/kg. Comparing with the same quantity of *Tripterygium hypoglaucum* (Levl.) Hutch, the Fengshiping had a higher inhibiting activity. Based on the data in table 3.1, the inhibiting activity of Fengshiping was 2.25 times higher than the *Tripterygium hypoglaucum* (Levl.) Hutch. The inhibiting activity of *Tripterygium hypoglaucum* (Levl.) Hutch. *Tripterygium hypoglaucum* (Levl.) Hutch alone in a dosage of 13.5g/kg was weaker than that of the Fengshiping containing 6g/kg *Tripterygium hypoglaucum* (Levl.) Hutch.

3.2 The effect of the Fengshiping on the humoral immunity in the AA mouse

The NIH mice, 20+2g weight, were injected with 0.05 ml FCA under the plantar skin of the right hindfoot. 3 weeks later the AA model mouse were obtained. The model mice were divided into 6 groups randomly and fed with the corresponding medicines for 5 days. At the beginning of the administration, all the mice were sensitized with 0.5ml 10% sheep RBC (SRBC). Five days later, all the mice were killed. Their spleens were taken out and washed by the Hank's solution to prepare the lymphocyte suspended solution. The concentration of the cells was adjusted to $2 \times 10^7$/ ml. 1 ml lymphocyte suspension, ml 0.2% SRBC and 1 ml 1:30 complement were added to one test tube. The tube was put in the water bath at 37°C for 1 hour. Then the tube was centrifuged at 2000rpm for 5 minutes. The supernatant fluid was separated and tested for its optical density at the 415nm wavelength on the 722 apeotrophotometer. The value was the represent of PFC quantity.

The other share of the blood samples got from the sensitized mice was separated the serum to test the potency of the antibody. The measured data were recorded on the way of Log2 value. (See the data in table 3.4)

**Table 3.4 The effect of Fengshiping on the humoral immunity in the mouse ($\bar{X} \pm S$)**

| group | dose (g/kg) | Mouse number | PFC (OD) | IgM(Log2) |
|---|---|---|---|---|
| control | - | 8 | 0.819 ± 0.013# | 6.875 ± 0.641 |
| AA model group | - | 10 | 0.940 ± 0.019** | 7.700 ± 0.599* |
| fengshiping | 5 | 8 | 0.834 ± 0.012**# | 6.875 ± 0.641# |
| fengshiping | 10 | 8 | 0.834 ± 0.012**# | 6.750 ± 0.886# |
| fengshiping | 20 | 8 | 0.830 ± 0.014**# | 6.375 ± 0.518# |
| Glucosidorum Tripterygll Totorum | 0.012 | 10 | 0.835 ± 0.015**# | 6.950 ± 0.597# |

Comparing with the control group *P<0.05, **P<0.01; comparing with the model group # P<0.05, ## P<0.01

According to the table 3.4, the levels of PFC and IgM in the AA mouse were higher than that of the normal mouse. The Fengshiping could lower the produce of the PFC and IgM in the AA mouse significantly.

Experimental example 4: The effect of the Fengshiping on the passive cutis anaphylactic reaction (PCA) in the rat

The rats were injected with the egg albumin at 10mg/kg in the muscle. At the same time, all the rats were injected with $2 \times 10^{10}$ (0.2 ml) *Bordetella pertussis* in the abdominal cavity for sensitization. 2 weeks later, all the rats were killed to sample the blood. All the blood samples were separated for preparing the serum.

60 rats, 150-200g, half male and half female, were divided into 6 groups at random. In the light narcosis condition induced by ether, each rat was shaved in the back and injected with the 2 concentrations of anti-egg-albumin serum 0.1ml under the skin at the shaved place. The serums were diluted at the ratios of 1:5(dl) and 1:10(d2) before the experiment. 48 hours later, all the rats were injected intravenously with the 0.5% Evans blue normal saline solution 1 ml which contains 1 mg egg albumin. 20 minutes later, the rats were sacrificed by decapitation. The rats' back skin were dissected and turned over. According to the degree and area of the blue stains exuded from the vessels, all the rats were evaluated by several people. The skin stained by the Evens blue were cut into pieces and soaked in 5ml 0.1% sodium sulfate acetone (7:3) solution for 48 hours. Then it was centrifuged to separate the supernatantsolution. The supernatants were measured for the optical density at the wavelength 590nm to calculate the degree of the PCA reaction and the inhibiting percent. The results were shown in table 4.

**Table 4 The effect of Fengshiping on the PCA in rat ($\bar{X} \pm S$)**

| Group | dose (g/kg) | value | | absorbency | |
|---|---|---|---|---|---|
| | | $d_1$ | $d_2$ | $d_1$ | $d_2$ |
| Control | - | 5.60 ± 1.78 | 2.40 ± 2.46 | 0.191 ± 0.129 | 0.096 ± 0.106 |
| Fengshiping | 12 | 7.50 ± 2.51 | 4.20 ± 2.49 | 0.402 ± 0.213* | 0.192 ± 0.175 |
| Fengshiping | 24 | 7.10 ± 2.13 | 4.10 ± 1.79 | 0.310 ± 0.177 | 0.137 ± 0.099 |
| Fengshiping | 48 | 6.00 ± 1.83 | 1.70 ± 1.95 | 0.121 ± 0.109 | 0.024 ± 0.026* |
| Tripterygium hypoglaucum (Levl.) Hutch. | 8 | 6.11 ± 1.27 | 2.56 ± 1.67 | 0.223 ± 0.122 | 0.074 ± 0.045 |
| Ketotifen | 0.1 | 2.78 ± 1.64** | 0.67 ± 1.41 | 0.033 ± 0.0244* | 0.027 ± 0.019* |

Comparing with the control group *P<0.05, **P<0.01

According to the table 4, it indicated that the Fengshiping had a weak effect on the PCA in the rat. Only on a high dosage, the inhibiting effect of Fengshiping was obviously different from that of the control group.

Experimental example 5: The effect of Fengshiping on the cytokines

5.1 The effect of Fengshiping on the levels of TNF a and IL-2 in the mouse.

60 ICR mice, 18-22g, half male and half female, were divided into 6 groups at random. Each group was fed with the corresponding medicines including the different dosages of Fengshiping and the other medicines. The medicines were administrated once a day for 10 days. 24 hours after the last administration, the mice were sampled the macrophage and spleen cells from the abdominal cavity in the aseptic condition. The samples were washed with Hank's solution for 2 times and non-serum RPIM 1640 medium for 1 time. Then the washed samples were diluted to the suspension with the 5% FCS-RPMI 1640 at the concentration of $2 \times 10^8$ / ml. Then the suspensions were added with 10ng/ml LPS or the 10ng/ml ConA and cultured in the 5% $CO_2$ condition for 48 hours at 37°C. Then the cultured suspension were measured for the TNF $\alpha$ and IL-2 levels with conventional methods.

The measurement of TNF $\alpha$

The plate was coated by mouse TNF-$\alpha$ monoclonal antibody. The plate was added with the cultured supernate on the dose of 50 $\mu$l/ well. Then the plate was put still for 60 minutes at the room temperature. Then the plate was added with biotin antibody mark at 25 °C for 2 hours. Then the enzyme labeled avidin was added into the plate for 30 minutes. After adding the substrate constant for 30 minutes, the plate was added with the stop solution. The mixed solution was measured for the OD value at the wavelength of the 450nm. The content of the TNF-$\alpha$ (ng/ml) was calculated on the data of OD value by the method of standard curve.

The measurement of the IL-2:

The CTLL cells which were on the logarithmic growth phase and whose growth depends on the IL-2, were adjusted to the suspension at the concentration of $1 \times 10^5$/ml with the 5% FCS-RPMI 1640. Then the 96 well cell culturing plate were added with the CTLL cell suspension on the quantity of 100 $\mu$l /well. The supernatant was added at the quantity of 100 $\mu$l /well and each sample was added to 3 wells. The samples cultured were compared with the different dilutions of standard rHIL-2 and the control sample (culture fluid) to measure the IL-2. All the samples were cultured in the 5% $CO_2$ for 24 hours at 37°C. 6 hours before the end of the culture, all the samples were centrifuged and the supernatant was separated. 110 $\mu$l supernatant was removed from each well and 10 $\mu$l MTT was added into each well. The samples were cultured for 3 hours at 37°C, and then the OD was measured at the wavelengths 570nm and 630nm. The final OD value of the sample was the difference of OD (570nm) and OD (630nm).

$$IL\text{-}2\,activity = \frac{\overline{SampleOD} - Control\,\overline{(Culture\,Fluid)OD}}{Standard\,Sample\,\overline{OD} - Control\,\overline{(Culture\,Fluid)OD}} \times activity\,of\,the\,standard\,sample\,(IU/ml)$$

**Table 5.1 The effect of Fengshiping on the TNF $\alpha$ nd IL-2 ($\bar{X} \pm S$)**

| group | dose (g/kg) | Mouse number | TNF (pg/ml) | IL-2 (IU/ml) |
|---|---|---|---|---|
| Control | - | 10 | 87.80 ± 14.63 | 26.30 ± 4.22 |
| | 12 | 10 | 62.14 ± 13.13** | 16.00 ± 2.89** |
| Fengshiping | 24 | 10 | 58.60 ± 9.63** | 18.80 ± 2.86** |
| | 36 | 10 | 54.40 ± 10.88** | 18.20 ± 2.86** |
| Tripterygium hypoglaucum (Levl.) Hutch. | 8 | 10 | 58.25 ± 10.32** | 16.00 ± 2.88** |
| cyclophosphane | 0.02 | 10 | 42.20 ± 9.57** | 10.10 ± 3.00** |
| *P<0.05, **P<0.01 | | | | |

According to the data in Table 5.1, it suggested that the Fengshiping have an obvious inhibiting effect on the TNF $\alpha$. On the dosage of 12g/kg, the medicine showed an obvious inhibiting effect. Along with the increase of the dosage, the inhibiting effect increased. But the dosage-effect curve went gently. The Fengshiping had an obvious inhibiting effect on the IL-2, but no dosage-effect relationship was observed.

5.2 The effect of Fengshiping on the IL-1, IL-6

70 NIH mice, 18-22g weight, half male and half female, were divided into 7 groups at random. All the groups were fed with the corresponding medicines (Fengshiping and the other medicines). The medicines were fed once a day for 10 days. 24 hours after the last administration, all the mice were killed and the macrophage and spleen cells from the abdominal cavity were sampled. The IL-1 and IL-6 in the samples were measured.

The measurement of IL-1:

The macrophages in the abdominal cavity were sampled in the asepsis condition. Then the samples were washed by the Hank's solution for 2 times and RPMI 1640 medium without serum for 1 time. Then the clear samples were adjusted to the $4 \times 10^6$ / ml cell suspension with 5% FCS-RPMI medium. 1 ml of the suspension was added to the test tube and cultured at 37°C for 1 hour. The non-adherent cells were abandoned. Then 5% FCS-RPMI 1640 and LPS (10 ng/ml) was added into the cell culture. The cells cultured in 5% $CO_2$ at 37°C for 72 hours. During the course, the cultured cells were frozen and thawed for several times. The final product was saved at 4°C. The C57 mice were sampled the thymus in the asepsis condition. Then the samples were prepared to the $1 \times 10^6$/ml cell suspension with 5% FCS-RPMI 1640.

100 $\mu$l supernatant separated from the frost thawing solution and 100 $\mu$l cell suspension of thymus were added into the 96-well flat bottom cell-culture plate. Each sample was cultured in 3 wells and compared with standard rHIL-1 at different dilutions and the control sample (culture fluid). Each well was added with 2ng ConA and then the plate was cultured in the 5% $CO_2$ at 37°C for 72 hours. 14 hours before the end of the culture, each well was added with 3H-TdR 0.1 $\mu$ Ci. The cultured cells were collected with multihead cell-harvesting apparatus and the cpm value was measured.

$$IL\text{-}1\,activity = \frac{\overline{Samplecpm} - Control\,\overline{(Culture\,Fluid)cpm}}{Standard\,Sample\,cpm - Control\,\overline{(Culture\,Fluid)cpm}} \times activity\,of\,the\,standard\,(ng/ml)$$

The measurement of the IL-6:

The spleen cells were sampled in the asepsis condition. Then the samples were washed by the Hank's solution for 2 times and non-serum RPMI 1640 medium for 1 time. Then the clear samples were adjusted to the $2 \times 10^6$/ml cell suspension with 5% FCS-RPMI medium. 1 ml of the suspension was added to the round-bottom centrifuge tube. After adding the ConA (1 ng/ml), the samples were cultured in the 5% $CO_2$ at 37°C for 72 hour.

The MH60 cells, which grew depending on the IL-6 and were on the logarithmic growth stage, were adjusted to the $1 \times 10^5$/ml cell suspension with the 5% FC-RPMI1640.

The 96-well flat bottom cell culturing plate was added with the MH60 cell suspension on the quantity of 100 μl/well and the culturing supernatant 25 μl /well. Then the fluid in each well was adjusted to the 200 μl with the 5% FCS-RPMI 1640. Each sample was cultured in three wells and compared with standard rHIL-6 at different dilutions and the pure culturing fluid. The plate was cultured in 5%$CO_2$ at 37°C for 72 hours. 6 hours before the end of the culture, the samples were centrifuged. 110 μl supernatant was removed from each well and 10 μl MTT was added into each well. The samples were kept at 37°C for 3 hours. And then they were measured for the OD at the wavelength 570nm and 630nm. The final OD value = OD 570nm - OD 630nm.

$$\text{IL-6 activity} = \frac{\text{SampleOD - Culturing Fluid ControlOD}}{\text{Standard SampleOD - Culturing Fluid ControlOD}} \times \text{Sample Dilution} \times \text{Activity Of The Standard (IU/ml)}$$

Table 5.2 The effect of Fengshiping on the IL-1, IL-6 ($\overline{X} \pm S$)

| Group | Dose (g/kg) | Mouse number | IL-1 (ng/ml) | IL-6 (IU/ml) |
|---|---|---|---|---|
| Control | - | 10 | 78.7 ± 7.1 | 94.6±6.8 |
| | 7.5 | 10 | 59.3 ± 4.9** | 64.9 ± 4.8** |
| | 15 | 10 | 53.3 ± 5.7** | 60.5 ± 4.3** |
| Fengshiping | 30 | 10 | 54.4 ± 4.8** | 56.0 ± 4.6** |
| | 60 | 10 | 47.0 ± 16.6** | 56.6 ± 6.1** |
| Tripterygium hypoglaucum (Levl.) Hutch. | 5 | 10 | 57.6 ± 4.7** | 65.7 ± 4.9** |
| cyclophosphane | 0.02 | 9 | 44.5 ± 7.7 | 49.6 ± 6.7** |

Based on the data in the table 5.2, the Fengshiping had an obvious inhibiting effect on the macrophage in producing of IL-1 and spleen cell in producing IL-6. Along with the increase of the dosage, the effect enhanced too.

5.3 The effect of Fenghsiping on the plasma NO in the AA rat

60 SD rats, 160 ~ 220g weight, half male and half female, were divided into 6 groups. The rats in the blank control group were injected the NS 0.5ml under the skin of the right hindfoot plantar skin. Other rats were injected with the FCA 0.5ml at the same place of the control group. 18 days later, the AA model was obtained. Then the rats were fed with the corresponding medicines or distilled water once a day for 5 days. 3 groups were fed with the solution of Fengshiping on the high, middle and low dilution. The positive group was fed with *Glucosidorum Tripterygll* Totorum. The blank control group and the model group were fed with the distilled water of the same volume. 1 hour after the last administration, each rat was sampled the blood from the abdominal aorta for 2 ml. The plasma of the blood samples were separated and saved at - 70 °C for the measurement. The measurement of NO was done on the direction of the NO reagent. 0.1 ml plasma was added in 0.6 ml reagent C and 0.4 ml double distilled water. After the mixture shaken up, it was added in 0.1ml reagent D and cultured on the ice for 60 min. Then it was centrifuged at 12000 rpm for 2 min. The supernatant was separated.0.6 ml supernatant was mixed with 0.4ml double distilled water and 0.1ml reagent A, and then it was cultured in the ice-water for 15 min. Then the mixture was added in reagent B 0.1 ml and put at the room temperature for 1 hour. Then the new mixture was measured for the OD at the wavelength 545nm. Based on the OD value of the sample, the content of NO was calculated on the standard curve. (See the result in table 5.3)

Table 5.3 The effect of Fengshiping on the plasma NO level in the AA rat($\overline{X}\pm S$)

| Group | Dose (g/kg) | Rat number | Content of NO (μ mol/L) | y (y=Lgx) |
|---|---|---|---|---|
| Control | | 8 | 13.55±1.11* | 1.131±0.032 |
| AA model | | 9 | 17.56±4.15 | 1.235±0.097 |

(continued)

| Group | Dose (g/kg) | Rat number | Content of NO (μ mol/L) | y (y=Lgx) |
|---|---|---|---|---|
| Fengshiping | 12 | 7 | $9.83 \pm 2.58^{**\triangle\triangle}$ | $0.985 \pm 0.087$ |
| Fengshiping | 24 | 7 | $10.12 \pm 1.56^{**\triangle\triangle}$ | $1.001 \pm 0.067$ |
| Fengshiping | 48 | 7 | $10.70 \pm 1.51^{**\triangle\triangle}$ | $1.026 \pm 0.062$ |
| Glucosidorum Tripterygll Totorum | 0.006 | 7 | $15.25 \pm 3.48$ | $1.173 \pm 0.099$ |

Comparing to the model group*P<0.05, **P<0.01; comparing to the *Glucosidorum Tripterygll* Totorum $\triangle\triangle$ P<0.01

Based on the data in table 5.3, the NO level was higher in the model group than in the blank control group. The Fengshiping had an obvious effect on lowering the NO level in the AA rat. The *Glucosidorum Tripterygll* Totorum had the similar effect but its effect was weaker than that of the Fengshiping.

Experimental example 6 The effect of Fengshiping on the T lymphocyte, $CD_4$, $CD_8$ and NK cells in the mouse

6.1 The effect of Fengshiping on the transform of lymphocyte in the normal mouse

80 NIH mice, half male and half female, were divided into 8 groups randomly and fed with the corresponding medicines once a day for 10 days. 24 hours after the last administration, all the mice were killed to sample the spleen cells aseptically. Then the samples were washed by the Hank's solution for 2 times and RPMI1640 medium without serum for 1 time. Then the clear samples were adjusted to the $2 \times 10^6$/ml cell suspension with 5% FCS-RPMI medium. The 96-well flat bottom cell culturing plate was added with the cell suspension on the quantity of 100 μl/well. Each sample was culturedin in three wells. 2ng ConA was added into two wells each as the stimulating reagent. The well without addition of ConA serves as the control well. The plate was cultured in 5% $CO_2$ at 37°C for 72 hours. 14 hours before the end of the culture, 3H-TdR 0.1 μ Ci was added into each well. The cells were harvested with the multihead cell harvesting instrument and measured for the cpm value. The average value was adopted as the sample's cpm value. The average value and the stimulating index of the different groups were compared directly. The stimulating index was calculated as follows:

$$\text{Stimulating Index} = \frac{\overline{\text{Stimulated cpm}}}{\overline{\text{Control cpm}}}$$

See the result in tale 6.1

Table 6.1 The effect of Fengshiping on the lymphacyto transformation induced by ConA in the mouse ($\overline{X} \pm S$)

| Group | Dose (g/kg) | Mouse number | cpm | Stimulating index |
|---|---|---|---|---|
| Control | | 10 | $20433 \pm 3579$ | $25.87 \pm 3.06$ |
| Fengshiping | 7.5 | 10 | $13566 \pm 1779^{**}$ | $27.29 \pm 7.67$ |
| | 15 | 10 | $12708 \pm 1692^{**}$ | $18.04 \pm 3.76$ |
| | 30 | 10 | $12809 \pm 2575^{**}$ | $16.17 \pm 4.37$ |
| | 60 | 10 | $12090 \pm 1706^{**}$ | $19.05 \pm 3.80$ |
| Hutch. | 2.5 | 10 | $18038 \pm 3359$ | $17.11 \pm 2.60$ |
| Tripterygium hypoglaucum (Levl.) | 5 | 10 | $12081 \pm 1039^{**}$ | $17.58 \pm 4.37$ |
| cyclophosphane | 0.02 | 9 | $9922 \pm 1145^{**}$ | $13.66 \pm 2.28$ |

Comparing to the control group*P<0.05, **P<0.01

According to the data in table 6.1, it indicated that the Fengshiping had an obvious inhibiting effect on the lymphocyte transformation and there was a dosage-effect relationship.

6.2 The effect of Fengshiping on the $CD_4$, $CD_8$ and NK cells

The experiment was same to 5.1. 24 hours after the last administration, the spleen cell samples were made into the $2 \times 10^8$/ml cell suspension with 5% FCS-RPIML640. The quantity of $CD_4$, $CD_8$, NK cells and the rate $CD_4/CD_8$

were measured with conventional method.

The measurement of CD$_4$ and CD8

The spleen cell suspension 50 $\mu$l was added on the glass slide to make the cell smear. The glass had been coated by the polylysine. The T cell of the mouse was set as the positive control sample. The cell smear was sealed with the serum of a normal mouse after it was fixed by the acetone. Then the sealed sample was added with the antibody of CD4 and CD$_8$ which were marked by the hominine biotin. It was incubated at 37°C for 2 hours. Then the sample was added with the avidin labeled by enzyme and put still for 10 min. After added with the substrate for 10 min, the mixed sample was washed and dyed with the hematoxylin for 2 min. Then the sample was dehydrated with the grade-alcohol and sealed with gelatin-glycerol. 200 cells in the smear were chosen as the research target under the high power microscope.

$$\text{Content Of Cell} = \frac{\text{Dyed cell number}}{200} \times 100\%$$

The measurement of the NK cell:

The preparation of the EC cell: The spleen cells were sampled in the asepsis condition. Then the samples were washed by the Hank's solution for 2 times and RPMI 1640 medium without serum for 1 time. Then the clear samples were adjusted to the $2 \times 10^8$/ml cell suspension with 5% FCS-RPMI medium. This cell suspension was used as the EC.
The preparation of the TC cell: The Yack-1 cells, which were sensitive to the mouse NK cell and on the logarithmic growth phase, were adjusted to the $4 \times 10^4$/ml cell suspension. It was the TC.
Measurement: EC and TC, 100 $\mu$l each were added in the 96-well flat bottom cell culturing plate. Each sample was cultured in three wells and set 2 control samples: EC and TC. (EC control: EC100 $\mu$l + 5% FCS RPMI 1640 100 $\mu$l; TC control: TC100 $\mu$l + 5% FCS RPML 1640 100 $\mu$l). The samples were cultured in 5% CO$_2$ at 37°C for 24 hours. 6 hours before the end of the culturing, the samples were centrifuged and 110 $\mu$l supernatant was removed from each well. And then 10 $\mu$l MTT was added into the wells. After incubation at 37°C for 3 hours, the mixed samples were measured for the OD value at the wavelength of 570 nm and 630 nm. The OD of each well = OD570nm - OD630nm.

$$\text{Activity Of NK} = \left(1 - \frac{\text{Sample } \overline{OD} - \text{EC Control } \overline{OD}}{\text{TC Control } \overline{OD}}\right) \times 100\%$$

**Table 6.2 The effect of Fengshiping on the CD4, CD8, NK cell ($\overline{X} \pm S$)**

| Group | Dose (g/kg) | Mouse number | CD4 (%) | CD8 (%) | CD4/CD8 | NK |
|---|---|---|---|---|---|---|
| Control | - | 10 | 20.80±2.94 | 14.80±2.49 | 1.42±0.18 | 40.13±4.89 |
| | 12 | 10 | 19.14±2.91 | 13.43±2.51 | 1.43±0.08 | 31.94±4.52**ΔΔ |
| Fengshiping | 24 | 10 | 17.30±2.51 ** | 12.00±2.40 | 1.46±0.16 | 35.36±3.40**ΔΔ |
| | 36 | 10 | 16.30±2.50** | 11.23+2.94** | 1.49±0.20 | 31.06±3.53**ΔΔ |
| Tripterygium hypoglaucum (Levl.) Hutch. | 8 | 10 | 16.25±2.25** | 11.50±2.45 | 1.44±0.18 | 32.20±2.00** |
| Cyclophosphane | 0.02 | 10 | 11.50±2.50** | 4.10±1.20** | 2.91±0.53** | 23.10±3.66** |

Comparing to the control group*P<0.05, **P<0.01; comparing to the cyclophosphane$^{\Delta\Delta}$P<0.01

According to the table 6.2, it was a relation between the dosage and the effect, but the dosage-effect curve was smooth. The effective dosage of Fengshiping on the inhibiting of Ca$_1$ was 24g/kg. The minimum effective dosage on inhibiting the CD$_8$ was 36g/kg. As the rate of CD$_4$/CD$_8$, the Fengshiping had no obvious effect. Cyclophosphane had an obvious effect on the inhibiting of the both kind of cells, and the inhibiting effect on the $CD_8$ was very powerful, which could increase the rate of $CD_4/CD_8$ magnificently.

As for NK cell, the Fengshiping had a remarkable inhibiting effect, but the dosage-effect relationship was not certain. As the same while, the cyclophosphane had shown an obvious inhibiting effect on the NK cell. On the dosage of 20mg/kg, the inhibiting effect of cyclophosphane was significantly different from that of the Fengshiping on the 3 dosages: 12, 24 and 36g / kg.

6.3 The effect on the transformation and function of the T lymphocyte in the AA mouse

NIH mice, 20 ± 2g weight, were injected with 0.05 ml FCA under the skin of the right hindfoot plantar skin to build the AA model. The mice in the control group were injected 0.05ml NS at the same place. 3 weeks later, after the AA model was obtained, all the mice were fed with the corresponding medicines once a day for 5 days. 5 days later, all the mice were sampled the blood to make the blood smear. The smears were dyed by the esterase. Then the smears were observed under the oil immersion lens to calculate the percent of the positive-dyed cells (it represented the content of the T cells in the blood). The mice were sampled the spleen cells in the condition of anesthesia and then the cell samples were prepared to the single cell suspension. The cell suspension was washed by PBS and then its supernatant were abandoned. The rest part was added with 4ml blood cytolysate . The mixed sample was shaken for 2 ~ 3 min to solve the RBC. After the RBCs were destroyed, the sample was centrifuged to separate and the supernatant was abandoned. The sample without supernatant was washed by the luminescence lotion for 2 times. Then it was centrifuged to separate and abandon the supernatant. In the next step, the sample was adjusted to the $1 \times 10^6$/ml cell suspension. 50 $\mu$l diluted antibody of $CD_4$ and $CD_8$ was added into each tube. Then the tubs were incubated at 4°C for 1 hour. After the culture, the samples were washed with the luminescence lotion for 2 times and added in the fixing fluid 2 ml. After fixing, the samples were filtrated through the 400-mesh screen to the FCA tube. The filtrated samples were analyzed by the flow cytometer (FCM). The result was shown in the table 6.3.

**Table 6.3 The effect of Fengshiping on the T cell in the AA mouse ($\overline{X} \pm S$)**

| Group | Dose (g/kg) | ANAE+ (%) | CD4+ (%) | CD8 (%) | CD4+/CD8+ |
|---|---|---|---|---|---|
| Control | - | 50.60±4.25 | 26.13±1.16 | 15.56±0.68 | 1.68±0.03 |
| AAmodel | - | 49.00±4.22▲ | 32.56±2.87** | 13.59±1.03** | 2.49±0.16** |
| | 7.5 | 49.13±4.03▲ | 27.30±1.76##▲ | 15.98±1.11##▲ | 1.71±0.04##▲ |
| Fengshiping | 15 | 49.31±3.29▲ | 27.96±1.67##▲ | 16.23±1.27##▲ | 1.73±0.05##▲ |
| | 30 | 48.56±3.23▲ | 26.75±1.94##▲ | 15.58±1.29##▲ | 1.72±0.04##▲ |
| Glucosidorum Tripterygll Totorum | 0.012 | 48.88±2.89▲ | 27.88±1.99##▲ | 16.33±1.31##▲ | 1.70±0.03##▲ |

n=8, comparing with the control group*P<0.05, **P<0.01; comparing with the model group# P<0.05, ## P<0.01; comparing with the control group▲P>0.05

According to the data in table 6.3, there was no significant difference in the different groups on the ANAE positive cell. But in AA mouse, the increase of the $CD_4$ was significant, while the decrease of $CD_8$ was significant too. So the rate of $CD_4/CD_8$ had a remarkable increase. The result indicated that the Fengshiping could adjust the $CD_4$, $CD_8$ and $CD_4/CD_8$ to the normal range.

Experimental example 7: The effect of Fengshiping on the phagocytic function of the macrophage in the mouse abdominal cavity

50 NIH mice, 18 ~ 22g weight, half male and half female, were divided into 5 groups and fed with the corresponding medicine solutions on the same volume. The administration was once a day for 7 days. 1 hour after the last administration, all the mice were injected with 0.2ml 10 % chick RBC into the abdominal cavity. 4 hours later, all the mice were killed and sampled the fluid in the abdominal cavity. The fluid samples were dropped on the glass and counted the number of the macrophage which had phagocytized the CRBC and the number of the CRBC in one macrophage. (See the result in table 7)

**Table 7 The effect of Fengshiping on the CRBC phagocytosis function of the macrophage in ICR mouse abdominal cavity ($\overline{X} \pm S$)**

| group | dose (g/kg) | Mouse number | Percent of phagocytosis (%) | phagocytosis index |
|---|---|---|---|---|
| Control | - | 10 | 25.75±9.40 | 1.28±0.20 |
| Fengshiping | 27 | 10 | 33.20±12.77 | 1.46±0.36 |
| Fengshiping | 40.5 | 10 | 35.20±10.16 | 1.21±0.20 |
| Fengshiping | 60.9 | 10 | 37.78±20.14 | 1.53±0.32 |
| dexamethasone | 0.005 | 10 | 8.33±10.13* | 1.10±0.18 |
| *P<0.05 | | | | |

According to the table 7, the Fengshiping had no obvious effect on the phagocytosis function of the macrophage in the mouse abdominal cavity.

Experimental example 8: The effect of Fengshiping on the hyperactivity of the capillary permeability in the mouse abdominal cavity

90 NIH mice, 18-22g weight, half male and half female, were divided into 9 groups and fed with the corresponding medicine solutions of the same volume. The medicines were fed once a day for 3 days or just 1 time. 1 hour after the last administration, each mouse were injected with 0.7% HAC - NS solution into the abdominal cavity. At the same time, each mouse was injected with the 0.5% Evans blue - NS solution into the vessel on the dose of 0.1ml/10 g. 30 min later; all the mice were killed by cervical disjoint. The abdominal cavity was opened and washed by the 5ml NS. The NS used was collected and adjusted to 8 ml the pure NS as the sample. The samples were centrifuged at 3000 rpm to get the supernatant. The supernatant was measured the OD at the wavelength at 590 nm. (See the result in table 8)

**Table 8 The effect of Fengshiping on the hyperfunction of the capillary permeability induced by the acetic acid in the mouse abdominal cavity ($\overline{X} \pm S$)**

| Group | Dose (g/kg) | Administration | Mouse number | Leakage of the tincture (OD) | P value |
|---|---|---|---|---|---|
| Control | - | - | 10 | 0.29±0.13 | |
| Fengshiping | 27 | qd×1 | 10 | 0.26±0.14 | >0.05 |
| Fengshiping | 40 | qd×1 | 10 | 0.25±0.10 | >0.05 |
| Fengshiping | 60 | qd×1 | 10 | 0.25±0.09 | >0.05 |
| Control | - | - | 10 | 0.28±0.15 | |
| Fengshiping | 27 | qd×3 | 10 | 0.25±0.12 | >0.05 |
| Fengshiping | 40 | qd×3 | 10 | 0.18±0.10 | <0.05 |
| Fengshiping | 60 | qd×3 | 10 | 0.15±0.13 | <0.05 |
| dexamethasone | 0.15 | qd×3 | 10 | 0.11±0.07 | <0.01 |

According to the data in table 8, it indicated that Fengshiping could obviously inhibit the hyperactivity of the capillary permeability induced by the acetic acid in the mouse abdominal cavity if it was fed for 3 days continuously. If the medicine was fed for just 1 time, the inhibiting effect was not obvious.

Experimental example 9: The effect of Fengshiping on the pleuritis exudation and the inflammatory cell aggregation induced by the carrageenan

The mice were divided into 5 groups at random and injected with 0.5% Evans blue NS solution into the caudal vein on the dosage of ml/10g. Then the mice were injected with the 0.03ml 1% carrageenan in the right chest cavity with the special syringe needle. 4 hours and 32 hours after the injection, the corresponding mice were killed and the abdominal cavity was cut open to expose the diaphragm. 2ml of the lotion were injected to the chest cavity by 2 times with a 1 ml injector. The lotion was collected and saved in a test tube. 20 μl of the lotion was collected and added into the 400 μl WBC dilution. The WBC in the mixed dilution was counted under the microscope. The rest of the lotion was centrifuged at 3000rpm for 10 min. The supernatant of the lotion was measured for the OD at the wavelength of 600nm. The OD value of the sample should be corrected with the corresponding OD value of the pure lotion. (See the result in table 9)

**Table 9 The effect of Fengshiping on the inflammatory cell aggregation induced by the carrageenan ($\overline{X} \pm S$)**

| Group | Dose (g/kg) | WBC number(2×105) | | Tincture exudation (OD) | |
|---|---|---|---|---|---|
| | | 4h | 32h | 4h | 32h |
| Control | - | 46.0±6.9 | 16.0±9.6 | 0.156±0.066 | 0.109±0.019 |
| Fengshiping | 27 | 26.8±4.5* | 14.2±8.0 | 0.121±0.062 | 0.116±0.031 |
| Fengshiping | 40.5 | 10.9±4.0** | 17.3 ± 4.6 | 0.100±0.048 | 0.153±0.032 |
| Fengshiping | 60 | 8.0±5.5** | 6.6±4.7* | 0.129±0.066 | 0.092±0.051 |
| dexamethasone | 0.05 | 12.7±10.2** | 4.4±4.0* | 0.085±0.045 | 0.063±0.017 |
| *P<0.05, **P<0.01 | | | | | |

According to the table 9, it indicated that the Fengshiping had an obvious inhibiting effect on the inflammatory cell aggregation. The effect was powerful at the early stage. The regression equation on the data of the fourth hour was as following: y=44.13 - 2.01x, r= - 0.9625. The effect on the late stage was weak. At the high dosage of 20g/kg, the medicine could affect the aggregation of the WBC. But it had no obvious effect on the pleuritis exudation

Experimental example 10: Effect on aggregation of leucocyte in rats' CMC sac

Sixty four SD rats, 150-180g weight, half male and half female, were randomly divided into 8 groups, which were fed with the same volume and different dosage of drug solution once a day, lasting 3 days. A day before experiment, rats were injected with 20ml 1% CMC solution into the sac at the rat's back caused by 20ml air injection before the experiment. 3.5 hour and 7.5 hour later, 0.1ml liquid in the sac was extracted each time, and was colored in 0.01% brilliant cresyl blue solution. Leucocyte was counted in the sac solution under microscope. The results are presented in the table 10.

**Table 10 effect on leucocyte counts of carboxymethyl cellulose sac of rats with Fengshiping ($\overline{X} \pm S$)**

| groups | dosage (g/kg) | rats number | WBC count(×107/L) | |
|---|---|---|---|---|
| | | | 3.5 hrs | 7.5 hrs |
| control | - | 8 | 9.7±4.2 | 57.7±17.3 |
| Fengshiping | 27×1 | 8 | 8.5±3.5 | 39.4±16.5 |
| Fengshiping | 40×1 | 8 | 8.7±7.3 | 35.3±23.2 |
| Fengshiping | 60×1 | 8 | 6.6±3.3 | 18.1±8.6** |
| Control | - | 8 | 10.97±6.7 | 35.6±11.2 |
| Fengshiping | 27×3 | 8 | 15.4±9.7 | 38.6±15.5 |
| Fengshiping | 40×3 | 8 | 4.8±3.4** | 18.4±12.2** |
| Fengshiping | 60×3 | 8 | 3.0±2.8** | 11.0±9.2* |
| cortisone | 0.1×3 | 8 | 14.2±8.0 | 41.7±16.0 |
| Control | - | 8 | 10.9±3.0 | 41.3±6.9 |
| Fengshiping | 18×7 | 8 | 6.2±3.0* | 11.4±6.4* |
| Fengshiping | 27×7 | 8 | 3.7±1.7** | 6.4±3.1** |
| Fengshiping | 40×7 | 8 | 2.5±1.9** | 5.9±3.9** |
| cortisone | 2mg×1 | 8 | 1.5±0.7** | 3.0±1.0** |
| Compared with control group**P<0.01 | | | | |

According to the table 10, the Fengshiping could inhibit significantly aggregation of leucocyte in the rats' CMC sac, and the inhibition showed apparent dosage-effect relation, which was stronger as administration time lasted. With administration of continuing seven days, wandering of leucocyte could be inhibited significantly at dosage of 18g/kg, at the same time, there was also very strong inhibition with cortisone injection into the sac.

Experimental example 11: The effect on croton oil-induced swelling in the ears of mice

60 NIH mice with weight of 18-22g, male and female accounting for half and half, were divided into 6 groups, which were fed with the same volume and different dosage of drug solution or tragacanth solution, once a day, lasting 3 days. 1 hour after the final administration, 2% croton oil mixture of 0.02ml was embrocated uniformly on the both sides of left ears of mice, and after 4 hours, the mice were snapped off its cervical vertebra and put to death. The left and right ears were cut down, then inflammatory and control ears were weighted by certain means. Difference of weight between left and right ears was the swelling extent of ears, results showing in table 11.

**Table 11 effect on croton oil-induced swelling of the ears of mice with Fengshiping ($\bar{X} \pm S$)**

| Groups | dosage (g/kg) | rats number | Degree of ears' swelling (mg) | inhibition rate (%) | P value |
|---|---|---|---|---|---|
| Control group | - | 10 | 44.38±9.40 | | |
| Fengshiping | 27 | 10 | 39.05±12.33 | 12.00 | >0.05 |
| Fengshiping | 40 | 10 | 36.65±5.83 | 17.64 | <0.05 |
| Fengshiping | 60 | 10 | 34.91±9.71 | 21.34 | <0.05 |
| dexamethasone | 0.003 | 10 | 14.13±5.75 | 68.16 | <0.01 |

It was seen from table 11, that Fengshiping had remarkable inhibition to croton oil-induced swelling of the ears of mice, and had quantity-effect relation, but which curve was gentle and smooth. There was significant inhibition effect at 13.5g/kg of dosage.

Experimental example 12: Effect on acetic acid-induced twisting reaction of mice

60 Kunming mice with weight of 18 ~ 22g, male and female accounting for half and half, were randomly divided into 6 groups, which were fed with different dosages of drug solution or Xihuangqi (i.e. tragacanth) solution. 1 hours after administration, 0.7% HAC saline of 0.2ml was injected, sc, and the mice were placed in a glass container and observed for the latent period before the twisting reaction of each mouse and the twisting times in 20 minutes, results are presented in table 12:

**Table 12 The effect of Fengshiping on acetic acid-induced twisting reaction of mice ($\bar{X} \pm S$)**

| groups | dosage (g/kg) | Rats numbers | Twisting times | Latent time (minute) |
|---|---|---|---|---|
| Control | - | 10 | 34.6±14.1 | 3.13±0.80 |
| Fengshiping | 27 | 10 | 28.2±5.76 | 3.82±0.85 |
| Fengshiping | 40 | 10 | 31.0±18.4 | 3.86±2.00 |
| Fengshiping | 60 | 10 | 20.7±12.3* | 3.95±1.42 |
| Tripterygium hypoglaucum (Levl.) Hutch. | 20 | 10 | 25.1±11.9 | 3.60±0.93 |
| morphine hydrochloride | 10mg/kg | 10 | 0.0±0.0 | 0.00±0.00 |

It was seen from table 12 that large dose of Fengshiping could delay the latent time before the HAC-induced twisting reaction and significantly reduce the twisting times in 20 minutes, which indicated Fengshiping had analgesic effects to some degree.

Experimental example 13: Effect on hemorheology of AA rats

Each of SD rats, 180 ± 20g weight, were injected intracutaneously with 0.05ml Freund's complete adjuvant on the right back foot metatarsal, and developed into adjuvant arthritis models. Each rats of negative control group were injected intracutaneously with 0.05ml saline on the right back foot metatarsal. Three weeks after obtaining the models, the rats were divided into model group, large, middle, small dosage group, negative control group and positive control group which were administered with Glucosidorum Tripterygll Totorum. The rats were fed once a day, lasting 5 days, 1 hours after administration for the last time, and 3ml blood was taken from abdominal aorta of rats and placed into test tube with 1% heparin as decoagulant, in which the whole blood viscosity was measured at shear rate of 230, 115, 46, 23, 11.5, 5.75S$^{-1}$ with NXE-1 cone and plate viscometer. The plasma viscosity was measured with WTP-BII adjustable constant pressure capillary viscometer. The haematocrit, erythrocyte aggregation index was measured with centrifugation method of packed cell volume. The rigidity index was calculated from the above-mentioned data. All the results showed in table 13.

**Table 13 Effect on hemorheology of adjutant arthritis model rats ($\overline{X} \pm S$)**

| Groups | Control group | Model group | Fengshiping (30g/kg) | Fengshiping (15g/kg) | Fengshiping (7.5g/kg) | Glucosidorum Tripterygll Totorum (6mg/kg) |
|---|---|---|---|---|---|---|
| whole blood viscosity (mPa.s) | | | | | | |
| 230S-1 | 4.43±0.09 | 4.92±0.15** | 4.56±0.09## | 4.49±0.11## | 4.54±0.16## | 4.66±0.28# |
| 115S-1 | 5.17±0.25 | 5.81±0.19** | 5.33±0.09## | 5.32±0.10## | 5.16±0.14## | 5.60±0.48# |
| 46S-1 | 6.84±0.11 | 7.20±0.18** | 6.56±0.13## | 6.59±0.09## | 6.67±0.14## | 6.70±0.48# |
| 23S-1 | 8.10±0.15 | 8.23±0.38 | 7.95±0.22 | 7.93±0.12 | 7.97±0.14 | 8.02±0.14 |
| 11.5S-1 | 9.35 ±0.08 | 9.78±0.10** | 9.40±0.08## | 9.45±0.10## | 9.30±0.133 | 9.31±0.12## |
| 6.5S-1 | 11.03±0.14 | 12.66±0.31** | 11.21±0.21## | 11.29±0.19## | 11.60±0.40## | 11.42±0.52## |
| Plasma viscosity (mPa.s) | 1.158±0.032 | 1.248±0.040** | 1.161±0.011## | 1.154+0.023## | 1.156±0.418## | 1.158±0.029## |
| corpuscular volume (%) | 46.13±2.31 | 41.33±1.12** | 45.10±2.39## | 44.33±1.52## | 45.71±1.04## | 46.03±3.59## |
| erythrocyte aggregation index | 2.49±0.032 | 2.58±0.083* | 2.46±0.066# | 2.49±0.094# | 2.44±0.048## | 2.45±0.091# |
| rigidity index | 6.155±0.536 | 7.127±0.557** | 6.506±0.558 | 6.525±0.146 | 6.394±0.200# | 6.621±0.883 |

Compared with negative control group*P<0.05, **P<0.01; compared with model control group# P<0.05, ##P<0.01.

According to the table 13, hemorheology of AA rats were changed significantly. The whole blood and plasma viscosity increased, haematocrit decreased, aggregation index and rigity index of erythrocyte increased. The Fengshiping could make the above-mentioned indices of hemorheology improv significantly.

Pharmacological effects of Fengshiping have been proved by the above-mentioned experiments. Many important pharmacological effects of Fengshiping had favorable dosage-effect relation, which implied the best therapeutic effectiveness might be obtained by adjusting the drug dosage at clinical work. The clinical studies on Fengshiping were carried on in China, Japan and Australia. These studies were operated according to international criterion related disease classification about diagnosis, therapy and curative effect. By using the Fengshiping capsules, its effective rate was around 94%, and its remarkable effective rate was around 60%. It could improve the symptoms such as morning stiffness, swelling and pain and so on and the related items. The results are presented in table 14-21.

**Table 14 Compared effect of treatment group with control group**

| Groups | Cases | remission (clinical recovery) | Notable effect | Effective | No effect | Notable effect rate (%) | Effective rate (%) |
|---|---|---|---|---|---|---|---|
| Treatment group | 32 | 5 | 14 | 11 | 2 | 59.38 | 93.74 |
| Control group | 30 | 3 | 10 | 12 | 5 | 43.33 | 83.33 |

**Table 15 Influence of IgG, IgA and Igm ($\overline{X} \pm S$)**

| Groups | cases | IgG | | IgA | | IgM | |
|---|---|---|---|---|---|---|---|
| | | pre- | post- | pre- | post- | pre- | post- |
| Normal | 32 | 12.45±1.48 | | 2.37±1.00 | | 1.58±0.59 | |
| Treatment group | 32 | 16.92±3.49 | 14.17±1.39** | 3.65±1.03 | 2.39±1.18** | 1.89±0.88 | 1.48±1.01 |
| Control | 30 | 17.03±4.12 | 15.14±2.21** | 3.45±1.86 | 2.32±1.75** | 2.03±0.95 | 1.76±1.28 |

Comparing with pre-treatment **P<0.01

**Table 16 Influence of C3 and C4($\overline{X}\pm S$)**

| groups | cases | C3 | | C4 | |
|---|---|---|---|---|---|
| | | pre - | post - | pre - | post - |
| normal group | 32 | 0.62±0.13 | | 0.14 ± 0.15 | |
| Treatment group | 32 | 1.88±0.72 | 1.25±0.66** | 0.48±0.12 | 0.26±0.06* |
| Control group | 30 | 2.13±0.64 | 1.56±0.62** | 0.40±0.16 | 0.25±0.07** |

Comparing with before therapy *P<0.05, **P<0.01

**Table 17 Influence of ESR and CRP ($\overline{X} \pm S$)**

| Groups | cases | ESR | | CRP | |
|---|---|---|---|---|---|
| | | pre- | post- | pre- | post- |
| Normal | 32 | 8.37±5.26 | | 4.12±1.88 | |
| Treatment | 32 | 66.58±9.01 | 30.31±6.53** | 13.35±6.67 | 8.86±3.34* |
| control | 30 | 73.33 ±9.09 | 35.83±11.61** | 14.21±6.29 | 9.04±3.15** |

Comparing with pre-treatment *P<0.05, **P<0.01.

**Table 18 Compared with power of gripping pre- and post-treatment ($\overline{X} \pm S$)**

| groups | Treatment Group | | Control Group | |
|---|---|---|---|---|
| | pre - | post - | pre - | post - |
| Gripping power of left hands (mmHg) | 39.13±20.24(15) | 80.47±34.61**(15) | 24.00± 17.63(21) | 55.155±23.27**(21) |
| Right hands | 35.85±22.46(15) | 85.32±36.32**(15) | 22.80±12.32(21) | 58.17±20.59**(21) |

Comparing with pre-treatment *P<0.05, **P<0.01

**Table 19 Influence of arthrosis swelling and pain and morning stiffness time ($\overline{X} \pm S$)**

| Items | Treatment Group | | Control Group | |
|---|---|---|---|---|
| | pre - | post - | pre - | post - |
| arthrosis swelling and pain | 5.79±0.52 | 3.14±0.83* | 5.56±2.15 | 3.92±0.26* |
| morning stiffness time (minute ) | 50.33±6.47 | 20.24±3.27** | 48.75±8.34 | 27.50±3.78** |

Comparing with pre-treatment *P<0.05,**P<0.01

**Table 20 Influence of RF changing to negative**

| Groups | Cases | RF negative | | |
|---|---|---|---|---|
| | | Pre - treatment | Post - treatment | Rate of negative turnaround (%) |
| Treatment group | 32 | 24 | 11 | 54.2 |
| Control group | 30 | 18 | 10 | 44.4 |

Fengshiping not only had significant effects, but also can make items such as SIL-2R, STNF, SIL-6R in plasma decrease, results are presented in the Table 21.

**Table 21 inflence of main indes such as SIL-2R, STNF $\alpha$ nd SIL-6R ($\overline{X}\pm S$)**

| groups | Cases | SIL - 2R(u/ml) | | STNF R1 (ng/ml) | | SIL - 6R(ng/ml) | |
|---|---|---|---|---|---|---|---|
| | | pre - | post - | pre - | post - | pre - | post - |
| Normal | 32 | 299 ± 68 (n=32) | | 1.56±0.48 (n=24) | | 72.05±18.26 (n=22) | |
| Fengshiping | 15 | 683±189 | 381±157** | 2.87±0.66 | 1.75±0.54** | 136.18±28.57 | 90.15±20.12** |
| Control | 10 | 765±203 | 412±167** | 2.63±0.72 | 2.38±0.39 (n=8) | 148.21±30.31 | 99.02±26.70** |

Comparing with pre-treatment **P<0.01

It was proved that the above-mentioned results on invention could be realized on the ways as following.

Practice example 1:

| | |
|---|---|
| *Epimedium brevicornum* Maxim. | 2222g |
| *Tripterygium hypoglaucum* (Levl.) Hutch. | 2222g |
| *Lycium barbarum* L. | 1111g |
| *Cuscuta chinensis* Lam. | 1111g |

*Tripterygium hypoglaucum* (Levl.) Hutch. was cut into pieces, extracted for three times with 13-, 10- and 10-fold water respectively, each time lasting 1 hour; *Epimedium brevicornum* Maxim was cut into segments, extracted three times with 15-, 10- and 10-fold water respectively, each extraction lasting 1 hour; *Lycium barbarum* L. was crushed to crude material, and immersed in 20-fold water at 80°C for 1 hour; *Cuscuta chinensis* Lam. was crushed to crude powder, immersed in 31-fold water at 80°C for 1 hour; decoction fluid or immersion fluid of four herbs were filtrated separately by means of passing through a macroporous adsorbent resin column, and eluted with 70% ethanol. When the color of effluent became deep significantly, collection of eluate was commenced; when the color of eluate became very weak, the collection was ended. Ethanol was recovered from the eluate of each herb. Then the eluate without alcohol was concentrated, dried to get the finally extracted drug powder; pharmaceutical grade of starch was blended with the four kinds of drug powder to 200g, mixed up uniformly and encapsulated into 1000 capsules. Each capsule which was prepared with the invented method was composed of 0.2g drugs extract and contained at least 2.0mg of icariine $C_{33}H_{40}O_{15}$. The regular dosage is: oral administration, three times every day, three capsules every time.

## Claims

1.   A pharmaceutical composition suitable for treating rheumatism, that is made from the following materials:

   *Tripterygium hypoglaucum* (Levl.) Hutch. in an amount of 1 to 4 parts by weight;
   *Epimedium brevicornum* Maxim. in an amount of 1 to 4 parts by weight;
   *Lycium barbarum* L. in an amount of 1 to 4 parts by weight; and
   *Cuscuta chinensis* Lam. in an amount of 1 to 4 parts by weight.

2.   A pharmaceutical composition as defined in claim 1, that is made from the following materials:

   *Tripterygium hypoglaucum* (Levl.) Hutch, in an amount of 2 parts by weight;
   *Epimedium brevicornum* Maxim. in an amount of 2 parts by weight;
   *Lycium barbarum* L. in an amount of 1 part by weight; and
   *Cuscuta chinensis* Lam. in an amount of 1 part by weight.

3.   A method for preparing a pharmaceutical composition as defined in claim 1 or claim 2, the method comprising:

   smashing the *Tripterygium hypoglaucum* (Levl.) Hutch. and the *Epimedium brevicornum* Maxim;
   decocting the resultant powders individually with water two to four times;
   soaking each of the *Lycium barbarum* L. and the *Cuscuta chinensis* Lam. in water at a temperature in the range of 80 °C to 95 °C one to three times;
   respectively;
   separately collecting and adding the decocted fluid and immersion fluid onto a corresponding macroporous adsorbent resin column;
   after the adsorption, washing the columns until the washing soluion turns clear; eluting the columns with a 60 % to 80 % alcohol;
   collecting the eluates while the colour turns from a deep colour to a very weak colour;
   pushing out the alcohol out of the upper part of the column with high pressure water and mixing the alcohol thereby displaced with the eluate;
   separately condensing all four eluates to a specific density of 1.10;
   dehydrating by spray drying each of the condensed eluates to get the extract of the crude herbs powders; and
   mixing the extracts uniformly.

4.   A method for preparing a pharmaceutical composition as defined in claim 1 or claim 2, the method comprising:

   cutting *Trypterygium hypoglaucum* (Levl.) Hutch and *Epimedium brevicornum* Maxim. into pieces;
   extracting the *Tripterygium hypoglaucum* (Levl.) Hutch., *Epimedium brevicornum* Maxim., *Lycium barbarum* L., and *Cuscuta chinesis* Lam., separately or together with a solution of 0 % to 95 % alcohol at a temperature in the range of 10°C to 98 °C one to four times;
   recovering the alcohol from the extracts separately or together;
   condensing, dehydrating and smashing the extracts and mixing the condensed, dehydrated, smashed extracts uniformly.

**5.** A method as defined in claim 4, further comprising the step of crushing the *Lycium barbarum* L., and *Cuscuta chinesis* Lam. before said extracting step.

**6.** A method as defined in claim 4 or claim 5, further comprising the step of mixing the extracts prior to said recovering step.

**7.** A method according to claim 3 or claim 4, further comprising the step of forming the pharmaceutical composition into a dosage form selected from the group consisting of a hard capsule, a soft capsule, a tablet, a granule, and an injection.

**8.** The use of a pharmaceutical composition as defined in claim 1 or claim 2 in the manufacture of a medicament for use in treating rheumatism.

**Patentansprüche**

**1.** Pharmazeutische Zusammensetzung, die sich zur Behandlung von Rheumatismus eignet, **dadurch gekennzeichnet, dass** sie aus den folgenden Materialien hergestellt wird:

*Tripterygium hypoglaucum* (Levl.) Hutch. in einer Menge von 1 bis 4 Gewichtsteilen;
*Epimedium brevicornum* Maxim. in einer Menge von 1 bis 4 Gewichtsteilen;
*Lycium barbarum* L. in einer Menge von 1 bis 4 Gewichtsteilen; und
*Cuscuta chinensis* Lam. in einer Menge von 1 bis 4 Gewichtsteilen.

**2.** Pharmazeutische Zusammensetzung nach Anspruch 1, die aus den folgenden Materialien hergestellt wird:

*Tripterygium hypoglaucum* (Levl.) Hutch. in einer Menge von 2 Gewichtsteilen;
*Epimedium brevicornum* Maxim. in einer Menge von 2 Gewichtsteilen;
*Lycium barbarum* L. in einer Menge von 1 Gewichtsteil; und
*Cuscuta chinensis* Lam., in einer Menge von 1 Gewichtsteil.

**3.** Verfahren zur Herstellung einer pharmazeutischen Zusammensetzung nach Anspruch 1 oder Anspruch 2, wobei das Verfahren umfasst:

Zerstampfen von *Tripterygium hypoglaucum* (Levl.) Hutch und *Epimedium brevicornum* Maxim;
zwei- bis viermaliges einzelnes Kochen der erhaltenen Pulver mit Wasser;
jeweils ein- bis dreimaliges Eintauchen von Lycium barbarum L. und *Cuscuta chinensis* Lam. in Wasser bei einer Temperatur im Bereich von 80°C bis 95°C ;
separates Sammeln und Auftragen der gekochten Flüssigkeit und Tauchflüssigkeit auf eine entsprechende Säule aus makroporösem Adsorptionsmittelharz;
nach der Adsorption Waschen der Säulen, bis die Waschlösung klar wird; Eluieren der Säulen mit 60% bis 80%igem Alkohol;
Sammeln der Eluate, während die Farbe von einer tiefen Farbe bis zu einer sehr schwachen Farbe wechselt;
Austreiben des Alkohols aus dem oberen Teil der Säule mit Wasser unter Hochdruck und Mischen des dadurch verdrängten Alkohols mit dem Eluat;
getrenntes Kondensieren aller vier Eluate bis zu einer spezifischen Dichte von 1.10;
Dehydratisieren durch Sprühtrocknen jedes der kondensierten Eluate, so dass man ein Extrakt der rohen Pflanzenpulver erhält; und gleichförmiges Mischen der Extrakte.

**4.** Verfahren zur Herstellung einer pharmazeutischen Zusammensetzung nach Anspruch 1 oder Anspruch 2, wobei das Verfahren umfasst:

Zerschneiden von *Tripterygium hypoglaucum* (Levl.) Hutch und *Epimedium brevicornum* Maxim in Stücke;
ein- bis viermaliges getrenntes oder gemeinsames Extrahieren von *Tripterygium hypoglaucum* (Levl.) Hutch, *Epimedium brevicornum* Maxim, *Lycium barbarum* L. und *Cuscuta chinensis* Lam. mit einer Lösung von 0% bis 95% Alkohol bei einer Temperatur im Bereich von 10°C bis 98°C.
getrenntes oder gemeinsames Gewinnen des Alkohols aus den Extrakten;
Kondensieren, Dehydratisieren, und Zerstampfen der Extrakte und gleichförmiges Mischen der kondensierten, dehydratisierten, zerstampften Extrakte.

**5.** Verfahren nach Anspruch 4, zudem umfassend den Schritt, bei dem *Lycium barbarum* L. und *Cuscuta chinensis* Lam. vor dem Extraktionsschritt zerkleinert werden.

**6.** Verfahren nach Anspruch 4 oder Anspruch 5, zudem umfassend den Schritt, bei dem die Extrakte vor dem Gewinnungsschritt gemischt werden.

**7.** Verfahren nach Anspruch 3 oder Anspruch 4, zudem umfassend den Schritt, bei dem die pharmazeutische Zusammensetzung zu einer Dosierungsform, ausgewählt aus der Gruppe, bestehend aus einer Hartkapsel, einer Weichkapsel, einer Tablette, einem Granulat und einer Injektion, geformt wird.

**8.** Verwendung der pharmazeutischen Zusammensetzung nach Anspruch 1 oder Anspruch 2 bei der Herstellung eines Medikaments zur Verwendung bei der Behandlung von Rheumatismus.

**Revendications**

**1.** Composition pharmaceutique adaptée au traitement des rhumatismes, qui est fabriquée à partir des substances suivantes :

*Tripterygium hypoglaucum* (Levl.) Hutch. à une teneur comprise entre 1 et 4 parts en masse ;
*Epimedium brevicornum* Maxim. à une teneur comprise entre 1 et 4 parts en masse ;
*Lycium barbarum* L. à une teneur comprise entre 1 et 4 parts en masse ; et
*Cuscuta chinensis* Lam. à une teneur comprise entre 1 et 4 parts en masse.

**2.** Composition pharmaceutique selon la revendication 1, qui est fabriquée à partir des substances suivantes :

*Tripterygium hypoglaucum* (Levl.) Hutch. à une teneur de 2 parts en masse ;
*Epimedium brevicornum* Maxim. à une teneur de 2 parts en masse ;
*Lycium barbarum* L. à une teneur de 1 part en masse ; et
*Cuscuta chinensis* Lam. à une teneur de 1 part en masse.

**3.** Procédé d'élaboration d'une composition pharmaceutique selon la revendication 1 ou la revendication 2, le procédé comprenant :

l'écrasement de *Tripterygium hypoglaucum* (Levl.) Hutch. et de *Epimedium brevicornum* Maxim ;
la décoction individuelle des poudres résultantes à l'eau deux à quatre fois ;
le trempage de chacune des substances *Lycium barbarum* L. et *Cuscuta chinensis* Lam. dans l'eau à une température comprise entre 80 °C et 95 °C une à trois fois ; respectivement ;
la récupération séparée et l'ajout du liquide de décoction et du liquide d'immersion sur une colonne de résine adsorbante macroporeuse adaptée ;
après l'adsorption, le lavage des colonnes jusqu'à ce que la solution de lavage devienne transparente ; l'élution des colonnes avec 60 % à 80 % d'alcool ;
la récupération des éluats tandis que la couleur passe d'une intensité profonde à une intensité très faible ;
l'élimination de l'alcool hors de la partie supérieure de la colonne à l'aide d'eau à pression élevée et le mélangeage de l'alcool ainsi déplacé avec l'éluat ;
la condensation séparée des quatre éluats jusqu'à une densité spécifique de 1,10 ;
la déshydratation par séchage par atomisation de chacun des éluats condensés pour obtenir l'extrait des poudres de plantes brutes ; et le mélangeage uniforme des extraits.

**4.** Procédé d'élaboration d'une composition pharmaceutique selon la revendication 1 ou la revendication 2, le procédé comprenant :

la découpe de *Tripterygium hypoglaucum* (Levl.) Hutch. et de *Epimedium brevicornum* Maxim. en morceaux ;
l'extraction de *Tripterygium hypoglaucum* (Levl.) Hutch., de *Epimedium brevicornum* Maxim., de *Lycium barbarum* L., et de *Cuscuta chinensis* Lam., séparément ou ensemble par une solution de 0 % à 95 % d'alcool à une température comprise entre 10 °C et 98 °C une à quatre fois ;

la récupération de l'alcool depuis les extraits séparément ou ensemble ;
la condensation, la déshydratation et l'écrasement des extraits et le mélangeage uniforme des extraits condensés, déshydratés et écrasés.

5. Procédé selon la revendication 4, comprenant en outre l'étape de broyage de *Lycium barbarum* L. et de *Cuscuta chinesis* Lam. avant ladite étape d'extraction.

6. Procédé selon la revendication 4 ou la revendication 5, comprenant en outre l'étape de mélangeage des extraits avant ladite étape de récupération.

7. Procédé selon la revendication 3 ou la revendication 4, comprenant en outre l'étape de mise en forme de la composition pharmaceutique en une forme galénique choisie dans le groupe constitué par une capsule dure, une capsule molle, un comprimé, un granule et une solution pour injection.

8. Utilisation d'une composition pharmaceutique selon la revendication 1 ou la revendication 2 dans la fabrication d'un médicament pour utilisation dans le traitement des rhumatismes.

**EP 1 510 217 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Chinese pharmacopoeia. 1995
- CHINESE PHARMACOPOEIA. 2000

- **ZHANG CUN.** A treatment of rheumatic arthritis with Tripterygium hypoglaucum. *HUNAN MAGAZINE OF TRADITIONAL CHINESE MEDICINE,* 1988, vol. 4 (4), 15-16